(19) Europäisches Patentamt
European Patent Office
Office européen des brevets

(11) Veröffentlichungsnummer: **0 130 318 A2**

(12) # EUROPÄISCHE PATENTANMELDUNG

(21) Anmeldenummer: **84105330.9**

(22) Anmeldetag: **14.11.81**

(51) Int. Cl.[4]: **C 07 D 249/08**, A 01 N 43/00

(30) Priorität: **28.11.80 DE 3044802**

(43) Veröffentlichungstag der Anmeldung: **09.01.85 Patentblatt 85/2**

(84) Benannte Vertragsstaaten: **AT BE CH DE FR GB IT LI NL SE**

(60) Veröffentlichungsnummer der früheren Anmeldung nach Art. 76 EPÜ: **0053311**

(71) Anmelder: **BAYER AG, Konzernverwaltung RP Patentabteilung, D-5090 Leverkusen 1 Bayerwerk (DE)**

(72) Erfinder: **Regel, Erik, Bergerheide 72a, D-5600 Wuppertal 1 (DE)**
Erfinder: **Büchel, Karl Heinz, Prof.Dr., Dabringhausener Strasse 42, D-5093 Burscheid (DE)**
Erfinder: **Lürssen, Klaus, Dr., August-Kierspel-Strasse 89, D-5060 Bergisch-Gladbach (DE)**
Erfinder: **Frohberger, Paul-Ernst, Dr., Willi-Baumeister-Strasse 5, D-5090 Leverkusen (DE)**

(54) **Substituierte 1-Phenyl-2-triazolyl-1-penten-3-ole, Verfahren zu ihrer Herstellung sowie ihre Verwendung als Pflanzenwachstumsregulatoren und Fungizide.**

(57) Neue substituierte 1-Phenyl-2-triazolyl-1-penten-3-ole der Formel

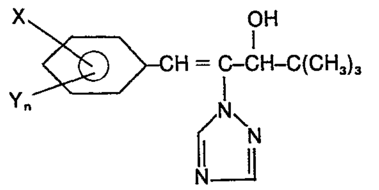

(I)

in welcher

X für 2-Trifluormethyl, 4-Trifluormethyl oder 3-Nitro steht,

Y für 2-Chlor, 3-Chlor oder 4-Chlor steht und

n für 0 oder 1 steht,

sowie deren 1,5-Naphthalin-disulfonsäure- und Chlorwasserstoff-Additionssalze,

ein Verfahren zur Herstellung der neuen Stoffe und deren Verwendung als Pflanzenwachstumsregulatoren und Fungizide.

EP 0 130 318 A2

Teilanmeldung zu der Anmeldung Nr. 81 109 686.6

BAYER AKTIENGESELLSCHAFT        5090 Leverkusen, Bayerwerk
Konzernverwaltung RP
Patentabteilung        Dü/bo/c

Substituierte 1-Phenyl-2-triazolyl-1-penten-3-ole, Verfahren zu ihrer Herstellung sowie ihre Verwendung als Pflanzenwachstumsregulatoren und Fungizide

Die vorliegende Erfindung betrifft neue substituierte 1-Phenyl-2-triazolyl-1-penten-3-ole, ein Verfahren zu ihrer Herstellung sowie ihre Verwendung als Pflanzenwachstumsregulatoren und Fungizide.

Es ist bereits bekannt geworden, daß bestimmte 4,4-Dimethyl-1-phenyl-2-triazolyl-1-penten-3-ole eine gute fungizide Wirksamkeit besitzen (vgl. DE-A 28 38 847). Die Wirkung dieser Verbindungen ist jedoch, insbesondere bei niedrigen Aufwandmengen und -konzentrationen, nicht immer voll befriedigend. Die pflanzenwachstumsregulierende Wirkung dieser Azol-Derivate ist ebenfalls nicht immer ganz befriedigend.

Weiterhin sind aus der GB-A 2 046 260 geometrische Isomere von bestimmten 1-Triazolyl-styrol-Derivaten mit pflanzenwuchsregulierenden und fungiziden Eigenschaften bekannt. Es werden in dieser Druckschrift jedoch keine

Verbindungen offenbart, in denen der Phenyl-Ring der Styrol-Einheit in der 2- oder 4-Position durch Trifluormethyl oder in der 3-Position durch Nitro substituiert ist.

Es wurden nun neue substituierte 1-Phenyl-2-triazolyl-1-penten-3-ole der Formel

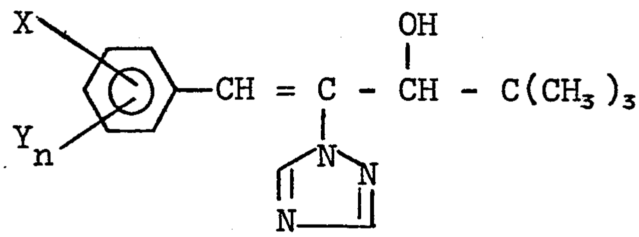

(I)

in welcher

X für 2-Trifluormethyl, 4-Trifluormethyl oder 3-Nitro steht,

Y für 2-Chlor, 3-Chlor oder 4-Chlor steht und

n für 0 oder 1 steht,

sowie deren 1,5-Naphthalin-disulfonsäure- und Chlorwasserstoff-Additionssalze gefunden.

Die erfindungsgemäßen Verbindungen der Formel (I) kommen in den geometrischen Isomeren E (trans) und Z (cis) vor.

Bei der E,Z-Nomenklatur werden die an der Doppelbindung stehenden Substituenten nach der Cahn-Ingold-Prelog-Regel nach abnehmender Priorität eingeordnet. Stehen die

bevorzugten Substituenten auf derselben Seite der Doppelbindung, liegt die Konfiguration Z (abgeleitet von zusammen) vor, stehen sie auf der entgegengesetzten Seite, liegt die Konfiguration E (abgeleitet von entgegen) vor.

Da außerdem ein asymmetrisches Kohlenstoffatom vorhanden ist, können die Verbindungen der Formel (I) in zwei optischen Isomerenformen vorkommen.

Die vorliegende Erfindung betrifft sowohl die einzelnen Isomeren als auch die Isomerengemische.

Weiterhin wurde gefunden, daß man die substituierten 1-Phenyl-2-triazolyl-1-penten-3-ole der Formel (I) erhält, wenn man 1-Phenyl-2-triazolyl-1-penten-3-one der Formel

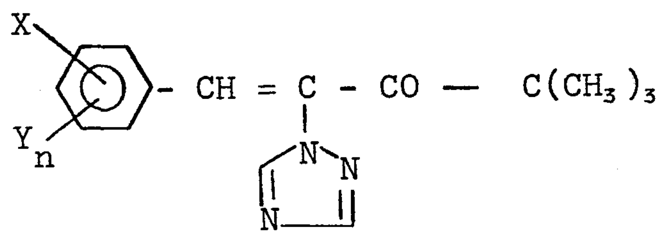

(II)

in welcher

X, Y und n die oben angegebene Bedeutung haben,

in allgemein üblicher Weise reduziert.

An die so erhaltenen Verbindungen der Formel (I) kann gegebenenfalls anschließend Chlorwasserstoff oder 1,5-Naphthalin-disulfonsäure addiert werden.

Schließlich wurde gefunden, daß die neuen substituierten 1-Phenyl-2-triazolyl-1-penten-3-ole der Formel (I) sowie deren Chlorwasserstoff- und 1,5-Naphthalin-disulfonsäure-Additionssalze starke pflanzenwachstumsregulierende und starke fungizide Eigenschaften aufweisen.

Überraschenderweise zeigen die erfindungsgemäßen Verbindungen eine bessere pflanzenwachstumsregulierende und fungizide Wirkung als die aus dem Stand der Technik bekannten 4,4-Dimethyl-1-phenyl-2-triazolyl-1-penten-3-ole, welche chemisch und wirkungsmäßig naheliegende Verbindungen sind (vgl. DE-A 28 38 847 und GB-A 2 046 260). Die erfindungsgemäßen Wirkstoffe stellen somit eine Bereicherung der Technik dar.

Verwendet man beispielsweise 4,4-Dimethyl-2-(1,2,4-triazol-1-yl)-1-(4-trifluormethylphenyl)-1-penten-3-on und Natriumborhydrid als Ausgangsstoffe, so kann der Ablauf des erfindungsgemäßen Verfahrens durch das folgende Formelschema wiedergegeben werden:

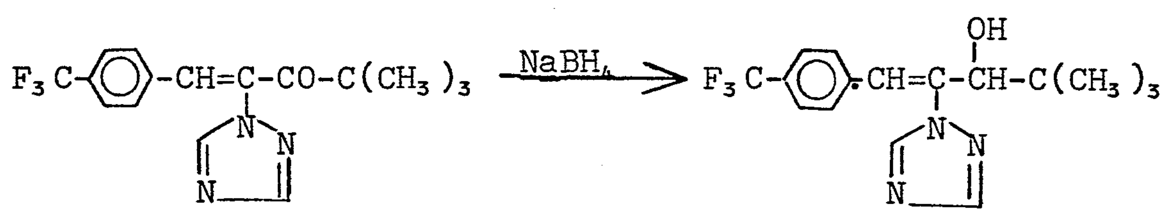


E/Z-Isomerengemisch E/Z-Isomerengemisch

Verwendet man beispielsweise 4,4-Dimethyl-2-(1,2,4-triazol-1-yl)-1-(2-trifluormethylphenyl)-1-penten-3-on und

Aluminiumisopropylat als Ausgangsstoffe, so kann der Ablauf des erfindungsgemäßen Verfahrens durch das folgende Formelschema wiedergegeben werden:

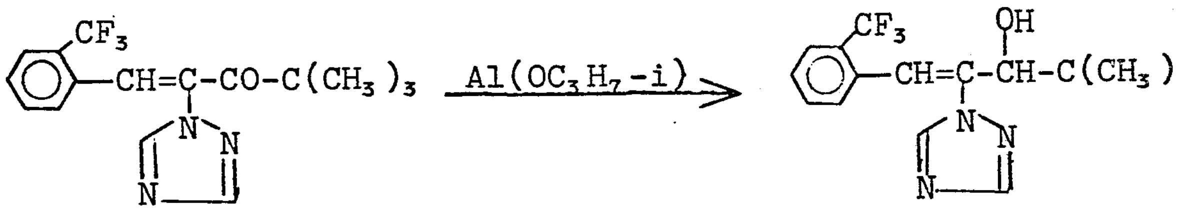


E/Z-Isomerengemisch Z-Isomeres

Die bei der Durchführung der erfindungsgemäßen Reduktion als Ausgangsstoffe benötigten 1-Phenyl-2-triazolyl-1-penten-3-one sind durch die Formel (II) definiert. In dieser Formel stehen X, Y und n für diejenigen Reste bzw. Zahlen, die bereits im Zusammenhang mit der Beschreibung der erfindungsgemäßen Stoffe der Formel (I) für diese Substituenten bzw. diesen Index genannt wurden.

Die 1-Phenyl-2-triazolyl-1-penten-3-one der Formel (II) sind noch nicht bekannt. Sie können jedoch in bekannter Art und Weise erhalten werden, indem man Triazolyl-pinakolin der Formel

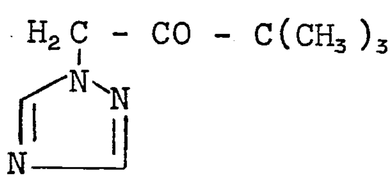

(III)

mit Aldehyden der Formel

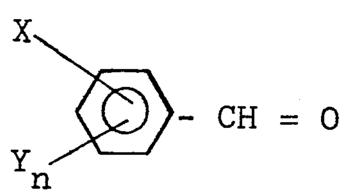

(IV)

in welcher

X, Y und n die oben angegebene Bedeutung haben,

in Gegenwart eines Lösungsmittels und in Gegenwart eines Katalysators umsetzt.

Als Lösungsmittel kommen für die Herstellung der 1-Phenyl-2-triazolyl-1-penten-3-one der Formel (II) vorzugsweise inerte organische Lösungsmittel in Frage. Hierzu gehören vorzugsweise Alkohole, wie Methanol und Ethanol; Ether, wie Tetrahydrofuran und Dioxan; aliphatische und cycloaliphatische Kohlenwasserstoffe, wie Hexan und Cyclohexan; aromatische Kohlenwasserstoffe, wie Benzol, Toluol und Cumol; halogenierte aliphatische und aromatische Kohlenwasserstoffe, wie Methylenchlorid, Tetrachlorkohlenstoff, Chloroform, Chlorbenzol und Dichlorbenzol.

Die Herstellung der Verbindungen der Formel (II) wird in Gegenwart eines Katalysators durchgeführt. Man kann alle üblicherweise verwendbaren sauren und insbesondere basischen Katalysatoren sowie deren Puffergemische einsetzen. Hierzu gehören vorzugsweise Lewis-Säuren, wie z.B. Bortrifluorid, Bortrichlorid, Zinntetrachlorid oder Titantetrachlorid; organische Basen wie Pyridin, 2,6-Dimethylmorpholin und Piperidin; sowie insbesondere Piperidinacetat.

Die Reaktionstemperaturen können bei der Durchführung dieses Verfahrens in einem größeren Bereich variiert

werden. Im allgemeinen arbeitet man zwischen 20 und 160°C, vorzugsweise bei der Siedetemperatur des jeweiligen Lösungsmittels.

Bei der Durchführung dieses Verfahrens setzt man auf 1 Mol Triazolylpinakolin der Formel (III) 1 bis 1,5 Mol Aldehyd der Formel (IV) und katalytische bis 0,2-molare Mengen an Katalysator ein. Die Produkte der Formel (II) fallen vorzugsweise als E/Z-Isomerengemische an. Eine Trennung in die reinen Isomeren ist auf übliche Art und Weise möglich, wie z.B. durch Kristallisation oder durch chromatographische Trennverfahren.

Die 1-Phenyl-2-triazolyl-1-penten-3-one der Formel (II) stellen allgemein interessante Zwischenprodukte dar, wie z.B. zur Herstellung der erfindungsgemäßen Verbindungen der Formel (I). In entsprechenden Konzentrationen zeigen sie auch wachstumsregulierende und fungizide Eigenschaften.

Die erfindungsgemäße Reduktion erfolgt in üblicher Weise, z.B. durch Umsetzung mit komplexen Hydriden, gegebenenfalls in Gegenwart eines Verdünnungsmittels, oder durch Umsetzung mit Aluminiumisopropylat in Gegenwart eines Verdünnungsmittels. Die Ausgangsstoffe der Formel (II) können dabei als E/Z-Isomerengemisch oder als reine Isomeren eingesetzt werden.

Arbeitet man mit komplexen Hydriden, so kommen als Verdünnungsmittel für die erfindungsgemäße Umsetzung polare

organische Lösungsmittel in Frage. Hierzu gehören vorzugsweise Alkohole, wie Methanol, Ethanol, Butanol, Isopropanol, und Ether, wie Diethylether oder Tetrahydrofuran. Die Reaktion wird im allgemeinen bei -10 bis +30°C, vorzugsweise bei -10 bis 20°C durchgeführt. Hierzu setzt man auf 1 Mol des Ketons der Formel (II) etwa 1 Mol eines komplexen Hydrids, wie Natriumborhydrid, Calciumborhydrid oder Lithiumalanat, ein. Die Isolierung der erfindungsgemäßen Verbindungen erfolgt in üblicher Weise, ebenso eine eventuelle Trennung der E/Z-Isomerengemische, die bei der Reduktion mit komplexen Hydriden immer entstehen, wenn man von E/Z-Isomerengemischen als Ausgangsprodukten der Formel (II) ausgeht.

Arbeitet man mit Aluminiumisopropylat, so kommen als Verdünnungsmittel für die erfindungsgemäße Umsetzung bevorzugt Alkohole, wie Isopropanol, oder inerte Kohlenwasserstoffe, wie Benzol, in Frage. Die Reaktionstemperaturen können wiederum in einem größeren Bereich variiert werden; im allgemeinen arbeitet man zwischen 20 und 120°C, vorzugsweise bei 50 bis 100°C.

Zur Durchführung der Reaktion setzt man auf 1 Mol des entsprechenden Ketons der Formel (II) etwa 1 bis 2 Mol Aluminiumisopropylat ein. Die Isolierung der erfindungsgemäßen Verbindungen erfolgt in üblicher Weise.

Bei der Reduktion mit Aluminiumisopropylat erhält man ausschließlich die Z-Isomeren.

Ein eindeutiges Charakterisierungsmerkmal für die beiden geometrischen Isomeren ist die $H^1$-Kernresonanz der zwei Triazol-Protonen. Die Differenz der Shiftwerte für diese beiden Protonen ist in den E-Formen ungefähr doppelt so groß wie in den entsprechenden Z-Formen.

Die Chlorwasserstoff- und 1,5-Naphthalin-disulfonsäure-Additionssalze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Art und Weise, z.B. durch Abfiltrieren, isoliert und gegebenenfalls durch Waschen mit einem inerten organischen Lösungsmittel gereinigt werden.

Die erfindungsgemäß verwendbaren Stoffe greifen in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanzen sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwachstumsregulierende Stoffe können z.B. zur Hemmung des vegetativen Wachstums der Pflanzen eingesetzt werden. Eine derartige Wuchshemmung ist u.a. bei Gräsern von wirtschaftlichem Interesse, denn dadurch kann die Häufigkeit des Grasschnittes in Ziergärten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, höhere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in stärkerem Maße der Blüten- und Fruchtbildung zugute kommen als den vegetativen Pflanzenteilen.

Die Wachstumsregulatoren läßt sich häufig auch eine Förderung des vegetativen Wachstums erzielen. Dies ist von großem Nutzen, wenn die vegetativen Pflanzenteile geerntet werden. Eine Förderung des vegetativen Wachstums kann aber auch gleichzeitig zu einer Förderung des generativen Wachstums führen, dadurch, daß mehr Assimilate gebildet werden, so daß mehr oder größere Früchte entstehen.

Ertragssteigerungen können in manchen Fällen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv

beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilität des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr unterschiedliche Erträge zu bringen. Schließlich ist es möglich, mit Wachstumsregulatoren zum Zeitpunkt der Ernte die zur Ablösung der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. dieses ist von besonderem Vorteil, weil sich dadurch eine optimale Anpassung an die Bedürfnisse des Marktes herbeiführen läßt. Weiterhin können Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z.B. Ananas oder Zierpflanzen

in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Saatgut mit Hilfe von Wachstumsregulatoren kann in frostgefährdeten Gebieten erwünscht sein, um Schädigungen durch Spätfröste zu vermeiden.

Schließlich kann mit Wachstumsregulatoren eine Resistenz der Pflanzen gegen Frost, Trockenheit oder hohen Salzgehalt des Bodens reduziert werden. Hierdurch wird die Kultivierung von Pflanzen in Gebieten möglich, die hierzu normalerweise ungeeignet sind.

Die erfindungsgemäßen Wirkstoffe weisen auch eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Als Pflanzenschutzmittel können die erfindungsgemäßen Wirkstoffe mit besonders gutem Erfolg zur Bekämpfung solcher Pilze eingesetzt werden, die echte Mehltauerkrankungen hervorrufen, so zur Bekämpfung von Erysiphe-Arten, wie z.B. gegen den Erreger des Gersten- bzw. des Getreidemehltaus (Erysiphe graminis); oder zur Bekämpfung von Venturia-Arten, wie z.B. gegen den Erreger des Apfelschorfs (Fusicladium dendriticum).

Hervorzuheben ist, daß die erfindungsgemäßen Stoffe nicht nur eine protektive Wirkung entfalten, sondern teilweise auch systemisch wirksam sind. So gelingt es, Pflanzen gegen Pilzbefall zu schützen, wenn man die Wirkstoffe über den Boden und die Wurzel oder über das Saatgut den oberirdischen Teilen der Pflanze zuführt.

Die Wirkstoffe können in die üblichen Formulierungen übergeführt werden, wie Lösungen, Emulsionen, Suspensionen, Pulver, Schäume, Pasten Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylenfettsäureester,

Polyoxyethylenfettalkoholether, z.B. Alkylarylpolyglykolether, Alkylsulfonate, Alkylsulfate, Arylsulfonate sowie Eiweißhydrolysate. Als Dispergiermittel kommen in Frage: z.B. Lignin-Sulfitablaugen und Methylcellulose. Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Die erfindungsgemäßen Wirkstoffe können in den Formulierungen in Mischung mit anderen Wirkstoffen vorliegen, wie Fungizide, Insektizide, Akarizide und Herbizide, sowie in Mischung mit Düngemitteln und anderen Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Art und Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder den Wirkstoff selbst in den Boden zu injizieren. Es kann auch das Saatgut der Pflanze behandelt werden.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Auch beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 Gew.-%. Bei der Saatgutbehandlung werden im allgemeinen Wirkstoffmengen von 0,001 bis 50 g je kg Saatgut, vorzugsweise 0,01 bis 10 g, benötigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Herstellungsbeispiele

Beispiel 1

(I-1)

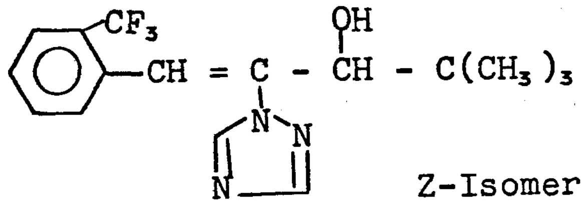

Z-Isomeres

(II-1)

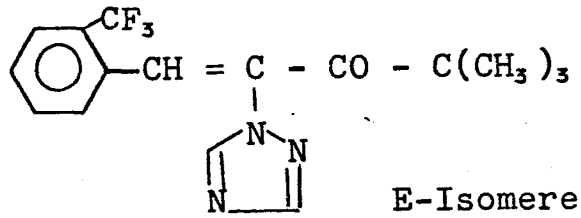

E-Isomeres

44,1 g (0,1365 Mol) 4,4-Dimethyl-2-(1,2,4-triazol-1-yl)-1-(2-trifluormethylphenyl)-1-penten-3-on als E/Z-Isomerengemisch und 27,9 g (0,1365 Mol) Aluminiumisopropylat werden 7 Stunden in 350 ml siedendem Isopropanol erhitzt; hierbei wird über eine 30 cm-Vigreux-Kolonne kontinuierlich Isopropanol und Aceton abdestilliert bis im Destillat kein Aceton mehr nachzuweisen ist. Danach wird die Lösung mit Eis/Salzsäure versetzt. Nach Extratkion mit Methylenchlorid wird die organische Phase über Natriumsulfat getrocknet, filtriert und im Vakuum eingedampft. Die verbleibende halbkristalline Masse wird über Kieselgel-60 (Merck)/Chloroform chromatographiert. Die ersten Fraktionen ergeben nach Abdampfen des Lösungsmittels 10,8 g (24 % d.Th.) E-isomeres 4,4-Dimethyl-2-(1,2,4-triazol-1-yl)-1-(2-trifluormethylphenyl)-1-penten-3-on vom Schmelzpunkt 82°C.

0130318

Die nächsten Fraktionen ergeben nach Abdampfen des Lösungsmittels 12,8 g (28 % der Theorie) Z-isomeres 4,4-Dimethyl-2-(1,2,4-triazol-1-yl)-1-(2-trifluormethylphenyl)-1-penten-3-ol vom Schmelzpunkt 136°C.

Herstellung des Ausgangsproduktes

(II-2) 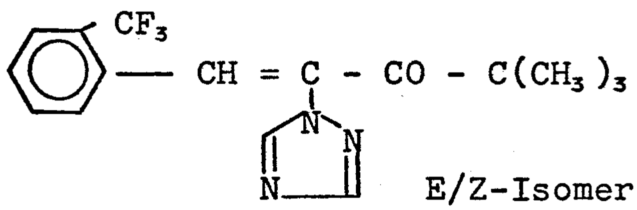

E/Z-Isomerengemisch

25,2 g (0,15 Mol) 3,3-Dimethyl-1-(1,2,4-triazol-1-yl)-butan-2-on und 26,1 g (0,15 Mol) 2-Trifluorbenzaldehyd werden in 350 ml Toluol mit 9 g Essigsäure und 3,6 ml Dimethylmorpholin 20 Stunden unter Rückfluß erhitzt, wobei das Reaktionswasser azeotrop entfernt wird. Die Toluollösung wird mit Wasser gewaschen, über Natriumsulfat getrocknet, filtriert und im Vakuum eingedampft. Man erhält 44,9 g (93 % der Theorie) 4,4-Dimethyl-2-(1,2,4-triazol-1-yl)-1-(2-trifluormethylphenyl)-1-penten-3-on als E/Z-Isomerengemisch vom Brechungsindex $n_D^{20}$ = 1,5113.

Beispiel 2

(I-2) 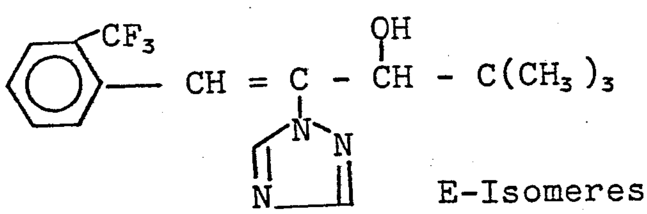

E-Isomeres

10,7 g (0,0345 Mol) E-isomeres 4,4-Dimethyl-2-(1,2,4-triazol-1-yl)-1-(2-trifluormethylphenyl)-1-penten-3-on (vgl. Beispiel 1) und 2,45 g (0,0233 Mol) Calciumchlorid werden in 150 ml Isopropanol bei -5°C mit einer Lösung von 0,85 g (0,0242 Mol) Natriumboranat in 20 ml Wasser tropfenweise versetzt.

Nach 18 Stunden werden 20 ml Aceton zugetropft. Nach Abdampfen des Lösungsmittels wird der verbleibende Rückstand in Wasser eingerührt und mit Methylenchlorid extrahiert. Die organische Phase wird abgetrennt, über Natriumsulfat getrocknet, filtriert und im Vakuum eingedampft. Die Kristallmasse wird mit Diisopropylether verrührt und abgesaugt. Man erhält 7,2 g (66,7 % der Theorie) E-isomeres 4,4-Dimethyl-2-(1,2,4-triazol-1-yl)-1-(2-trifluormethylphenyl)-1-penten-3-ol vom Schmelzpunkt 165°C.

Beispiel 3

(I-3) 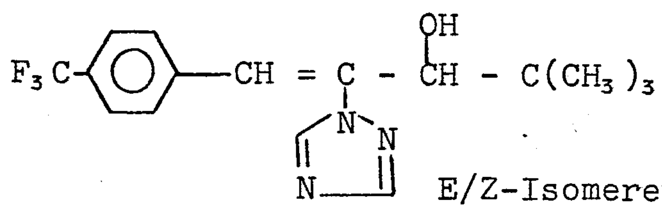

E/Z-Isomerengemisch

61,5 g (0,19 Mol) 4,4-Dimethyl-2-(1,2,4-triazol-1-yl)-1-(4-trifluormethylphenyl)-1-penten-3-on als E/Z-Isomerengemisch und 14,1 g (0,1275 Mol) Calciumchlorid werden in 400 ml Isopropanol bei -5°C mit einer Lösung von 5,05 g (0,13 Mol) Natriumboranat in 100 ml Wasser tropfenweise versetzt. Nach 15 Stunden werden 60 ml Aceton

zugetropft. Nach Abdampfen des Lösungsmittels wird der verbleibende Rückstand in Wasser eingerührt und mit Essigsäureethylester extrahiert. Die Lösung wird über Natriumsulfat getrocknet, filtriert und im Vakuum eingedampft. Man erhält 60,3 g (97 % der Theorie) 4,4-Dimethyl-2-(1,2,4-triazol-1-yl)-1-(4-trifluormethylphenyl)-1-penten-3-ol als E/Z-Isomerengemisch vom Schmelzpunkt 54 - 55°C.

In entsprechender Weise werden die folgenden Verbindungen der allgemeinen Formel (I) erhalten:

Tabelle 1

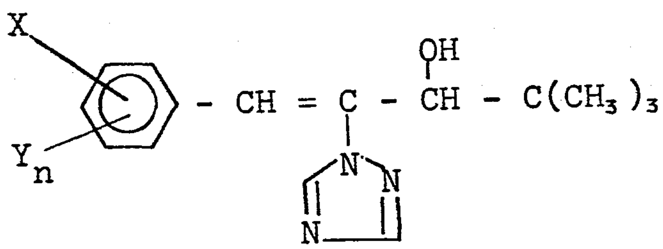

(I)

| Bsp.-Nr. | X | $Y_n$ | Schmelzpunkt (°C) |
|---|---|---|---|
| I-4 | 4-$CF_3$ | 2-Cl | 142 (Z-Isomeres) |
| I-5 | 4-$CF_3$ | 2-Cl | 134 (E-Isomeres) |
| I-6 | 4-$CF_3$ | - | 128 (Z-Isomeres) |
| I-7 | 4-$CF_3$ | - | 130 (E-Isomeres) |
| I-8 | 4-$CF_3$ | 3-Cl | 130 (Z-Isomeres) |
| I-9 | 4-$CF_3$ | 3-Cl | 141 (E-Isomeres) |
| I-10 | 3-$NO_2$ | 4-Cl | 150 (Z-Isomeres) |
| I-11 | 3-$NO_2$ | 4-Cl | Harz (E-Isomeres) |

Entsprechend Beispiel 1 und entsprechend dem im Text beschriebenen Verfahren werden die folgenden Ausgangsstoffe der Formel (II) erhalten:

Tabelle 2

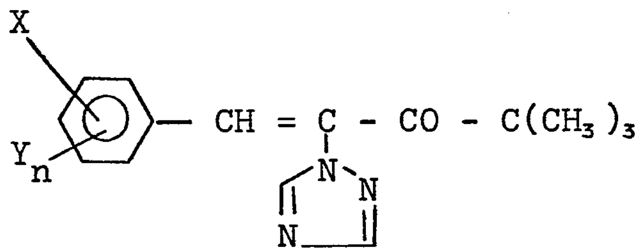

(II)

| Bsp.-Nr. | X | $Y_n$ | Schmelzpunkt (°C) bzw. Brechungsindex ($n_D^{20}$) |
|---|---|---|---|
| II-3 | 4-$CF_3$ | - | 119 (E-Isomeres) |
| II-4 | 4-$CF_3$ | 2-Cl | 1,5134 (E/Z-Gemisch) |
| II-5 | 4-$CF_3$ | 3-Cl | 1,5236 (E/Z-Gemisch) |

Anwendungsbeispiele

In den nachfolgenden Anwendungsbeispielen werden die nachstehend angegebenen Verbindungen als Vergleichssubstanzen eingesetzt:

(A) = 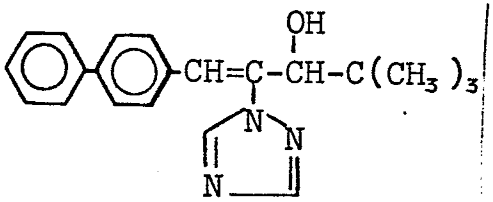

bekannt aus:
DE-A 29 20 437, Verb. 3
GB-A 2 046 260; Verb. 6
GB-A 2 004 276; Verb. 12
(= DE-A 28 38 847)

(B) = 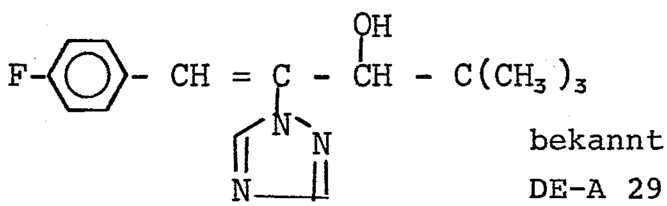

bekannt aus:
DE-A 29 20 437; Verb. 2
GB-A 2 046 260; Verb. 4
GB-A 2 004 276; Verb. 15
(= DE-A 28 38 847)

(C) = 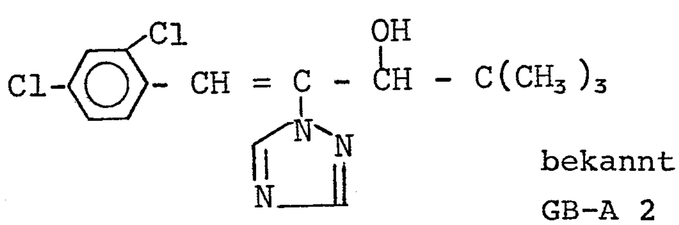

bekannt aus:
GB-A 2 046 260; Verb. 2
GB-A 2 004 276, Verb. 14
(= DE-A 28 38 847)

Beispiel A

Wuchshemmung bei Gras (Festuca pratensis)

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gras (Festuca pratensis) wird im Gewächshaus bis zu einer Wuchshöhe von 5 cm angezogen. In diesem Stadium werden die Pflanzen mit den Wirkstoffzubereitungen tropfnaß besprüht. Nach 3 Wochen wird der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die Wirkstoffe gemäß Beispielen (I-3), (I-5) und (I-7) zeigen in diesem Test eine bessere Wuchshemmung als die aus dem Stand der Technik bekannten Verbindungen (A), (B) und (C).

0130318

Beispiel B

Wuchshemmung bei Gerste

| | | |
|---|---|---|
| Lösungsmittel: | 30 Gewichtsteile | Dimethylformamid |
| Emulgator: | 1 Gewichtsteil | Polyoxyethylen-Sorbitan-Monolaurat |

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Gerstepflanzen werden im Gewächshaus bis zum 2-Blattstadium angezogen. In diesem Stadium werden die Pflanzen tropfnaß mit den Wirkstoffzubereitungen besprüht. Nach 3 Wochen wird bei allen Pflanzen der Zuwachs gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Der Wirkstoff gemäß Beispiel (I-7) zeigt in diesem Test eine bessere Wuchshemmung als die aus dem Stand der Technik bekannten Verbindungen (A), (B) und (C).

Beispiel C

Wuchshemmung bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der Zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die Wirkstoffe gemäß Beispielen (I-4), (I-5), (I-6) und (I-7) zeigen in diesem Test eine bessere Wuchshemmung als die aus dem Stand der Technik bekannten Verbindungen (A), (B) und (C).

## Beispiel D

### Wuchshemmung bei Sojabohnen

Lösungsmittel: 10 Gewichtsteile Dimethylformamid
Emulgator: 2 Gewichtsteile Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnenpflanzen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Die Wirkstoffe gemäß Beispielen (I-3), (I-4), (I-5), (I-6) und (I-7) zeigen in diesem Test eine bessere Wuchshemmung als die aus dem Stand der Technik bekannte Verbindung (A).

## Beispiel E

### Wuchshemmung bei Zuckerrüben

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen Ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchsbeeinflussung in Prozent des Zuwachses der Kontrollpflanzen berechnet. Es bedeuten 0 % Wuchsbeeinflussung ein Wachstum entsprechend dem der Kontrollpflanzen. 100 % Wuchshemmung bedeutet Stillstand des Wachstums.

Die Wirkstoffe gemäß Beispielen (I-3), (I-4), (I-5), (I-6) und (I-7) zeigen in diesem Test eine bessere Wuchshemmung als die aus dem Stand der Technik bekannten Verbindungen (A) und (B).

## Beispiel F

### Ethylenbildung

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Aus Sojabohnenblättern werden Blattstücke gleicher Größe gestanzt. Diese werden zusammen mit 1 ml Wirkstoffzubereitung bzw. Kontrollösung in luftdicht verschließbare Gefäße gegeben. Nach 24 Stunden wird das Ethylen, das sich in den Gefäßen gesammelt hat, mit üblichen Nachweismethoden bestimmt. Die Ethylenentwicklung der mit den Wirkstoffzubereitungen behandelten Blattstücke wird mit derjenigen der Kontrollen verglichen.

Es bedeuten:

0 Ethylenentwicklung wie bei der Kontrolle
\+ leicht erhöhte Ethylenentwicklung
++ stark erhöhte Ethylenentwicklung
+++ sehr stark erhöhte Ethylenentwicklung

Der Wirkstoff gemäß Beispiel (I-3) verursacht in diesem Test eine höhere Ethylenbildung als die aus dem Stand der Technik bekannten Verbindungen (A) und (B).

0130318

## Beispiel G

Stimulierung der Photosynthese

Nach Behandlung von Sojabohnenpflanzen mit dem Wirkstoff gemäß Herstellungsbeispiel (I-4) wurde eine Stimulierung der Photosynthese gegenüber Kontrollpflanzen festgestellt. Demgegenüber brachte die Behandlung mit der bekannten Verbindung (A) diesen Effekt nicht.

Beispiel H

Erysiphe-Test (Gerste) / protektiv

Lösungsmittel: 100 Gewichtsteile Dimethylformamid
Emulgator: 0,25 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit Sporen von Erysiphe graminis f.sp.hordei bestäubt.

Die Pflanzen werden in einem Gewächshaus bei einer Temperatur von ca. 20°C und einer relativen Luftfeuchtigkeit von ca. 80 % aufgestellt, um die Entwicklung von Mehltaupusteln zu begünstigen.

7 Tage nach der Inokulation erfolgt die Auswertung.

Eine deutliche Überlegenheit in der Wirksamkeit gegenüber der aus dem Stand der Technik bekannten Verbindung (A) zeigen bei diesem Test z.B. die Verbindungen gemäß folgender Herstellungsbeispiele: (I-3), (I-4), (I-5) und (I-7).

Beispiel I

Gerstenmehltau-Test (Erysiphe graminis var. hordei) / systemisch (pilzliche Getreidesproßkrankheit)

Die Anwendung der Wirkstoffe erfolgt als pulverförmige Saatgutbehandlungsmittel. Sie werden hergestellt durch Abstrecken des Wirkstoffes mit einem Gemisch aus gleichen Gewichtsteilen Talkum und Kieselgur zu einer feinpulverigen Mischung mit der gewünschten Wirkstoffkonzentration.

Zur Saatgutbehandlung schüttelt man Gerstensaatgut mit dem abgestreckten Wirkstoff in einer verschlossenen Glasflasche. Das Saatgut sät man mit 3 x 12 Korn in Blumentöpfe 2 cm tief in ein Gemisch aus einem Volumenteil Fruhstorfer Einheitserde und einem Volumenteil Quarzsand ein. Die Keimung und der Auflauf erfolgen unter günstigen Bedingungen im Gewächshaus. 7 Tage nach der Aussaat, wenn die Gerstenpflanzen ihr erstes Blatt entfaltet haben, werden sie mit frischen Sporen von Erysiphe graminis var. hordei bestäubt und bei 21 - 22°C und 80 - 90 % rel. Luftfeuchte und 16-stündiger Belichtung weiter kultiviert. Innerhalb von 6 Tagen bilden sich an den Blättern die typischen Mehltaupusteln aus.

Der Befallsgrad wird in Prozent des Befalls der unbehandelten Kontrollpflanzen ausgedrückt. So bedeutet 0 % keinen Befall und 100 % den gleichen Befallsgrad wie bei der unbehandelten Kontrolle. Der Wirkstoff ist um so wirksamer je geringer der Mehltaubefall ist.

Eine deutliche Überlegenheit in der Wirksamkeit gegenüber den aus dem Stand der Technik bekannten Verbindungen (A) und (C) zeigt in diesem Test z.B. die Verbindung gemäß Herstellungsbeispiel (I-3).

Beispiel J

Saatgutbeizmittel-Test / Streifenkrankheit der Gerste (samenbürtige Mykose)

Zur Herstellung eines zweckmäßigen Trockenbeizmittels verstreckt man den Wirkstoff mit einem Gemisch aus gleichen Gewichtsteilen Talkum und Kieselgur zu einer feinpulverigen Mischung mit der gewünschten Wirkstoffkonzentration.

Zur Beizung schüttelt man Gerstensaatgut, das durch Drechslera graminea (Syn. Helminthosporium gramineum) natürlich verseucht ist, mit dem Beizmittel in einer verschlossenen Glasflasche. Das Saatgut setzt man auf feuchten Filterscheiben in verschlossenen Petrischalen im Kühlschrank 10 Tage lang einer Temperatur von 4°C aus. Dabei wird die Keimung der Gerste und gegebenenfalls auch der Pilzsporen eingeleitet. Anschließend sät man die vorgekeimte Gerste mit 2 x 50 Korn 3 cm tief in Frühstorfer Einheitserde und kultiviert sie im Gewächshaus bei Temperaturen um 18°C in Saatkästen, die täglich16 Stunden dem Licht ausgesetzt werden. Innerhalb von 3 bis 4 Wochen bilden sich die typischen Symtome der Streifenkrankheit aus.

Nach dieser Zeit bestimmt man die Anzahl der kranken Pflanzen in Prozent der insgesamt aufgelaufenen Pflanzen. Der Wirkstoff ist umso wirksamer je weniger Pflanzen erkrankt sind.

Eine deutliche Überlegenheit in der Wirksamkeit gegenüber der aus dem Stand der Technik bekannten Verbindung (A) zeigt bei diesem Test z.B. die Verbindung gemäß folgendem Herstellungsbeispiel: (I-3).

Patentansprüche

1) Substituierte 1-Phenyl-2-triazolyl-1-penten-3-ole der Formel

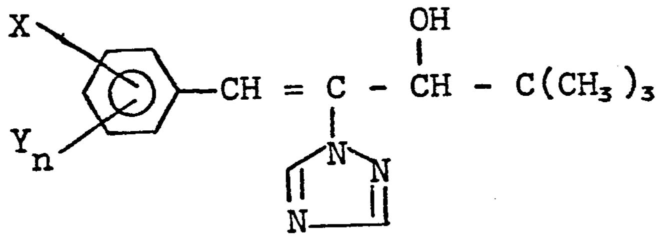

(I)

in welcher

X für 2-Trifluormethyl, 4-Trifluormethyl oder 3-Nitro steht,

Y für 2-Chlor, 3-Chlor oder 4-Chlor steht und

n für 0 oder 1 steht,

sowie deren 1,5-Naphthalin-disulfonsäure- und Chlorwasserstoff-Additionssalze.

2) Verfahren zur Herstellung von substituierten 1-Phenyl-2-triazolyl-1-penten-3-olen der Formel

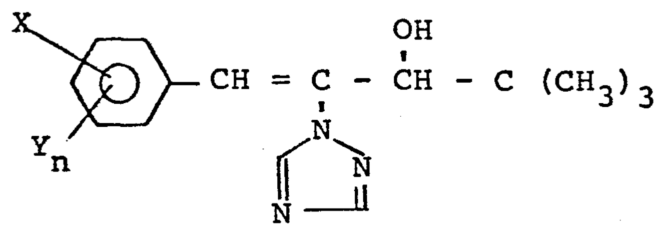

(I)

in welcher

X für 2-Trifluormethyl, 4-Trifluormethyl oder 3-Nitro steht,

Y für 2-Chlor, 3-Chlor oder 4-Chlor steht und

n für 0 oder 1 steht,

sowie von deren 1,5-Naphthalin-disulfonsäure- und Chlorwasserstoff-Additionssalzen, dadurch gekennzeichnet, daß man 1-Phenyl-2-triazolyl-1-penten-3-one der Formel

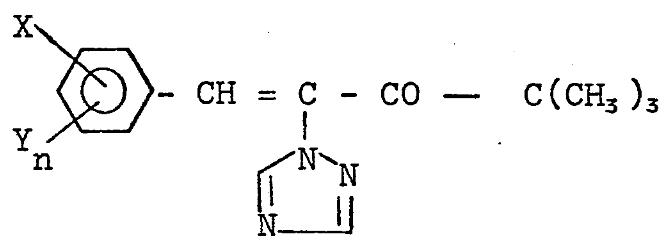
(II)

in welcher

X, Y und n die oben angegebene Bedeutung haben,

in allgemein üblicher Weise reduziert und gegebenenfalls anschließend an die erhaltenen Verbindungen der Formel (I) 1,5-Naphthalin-disulfonsäure oder Chlorwasserstoff addiert.

3) Pflanzenwachstumsregulierende und fungizide Mittel, gekennzeichnet durch einen Gehalt an mindestens einem substituierten 1-Phenyl-2-triazolyl-1-penten-3-ol der Formel (I) bzw. einem 1,5-Naphthalin-disulfonsäure- oder Chlorwasserstoff-Additionssalz eines substituierten 1-Phenyl-2-triazolyl-1-penten-3-ols der Formel (I).

4) Verfahren zur Regulierung des Pflanzenwachstums sowie zur Bekämpfung von Pilzen, dadurch gekennzeichnet, daß man substituierte 1-Phenyl-2-triazolyl-1-penten-3-ole der Formel (I) bzw. deren 1,5-Naphthalin-disulfonsäure-oder Chlorwasserstoff-Additionssalze auf Pflanzen und/oder ihren Lebensraum ausbringt.

5) Verwendung von substituierten 1-Phenyl-2-triazolyl-1-penten-3-olen der Formel (I) bzw. von 1,5-Naphthalin-disulfonsäure-oder Chlorwasserstoff-Additionssalzen von substituierten 1-Phenyl-2-triazolyl-1-penten-3-olen der Formel (I) zur Regulierung des Pflanzenwachstums bzw. zur Bekämpfung von Pilzen.

6) Verfahren zur Herstellung von pflanzenwachstumsregulierenden und fungiziden Mitteln, dadurch gekennzeichnet, daß man substituierte 1-Phenyl-2-triazolyl-1-penten-3-ole der Formel (I) bzw. 1,5-Naphthalin-disulfonsäure- oder Chlorwasserstoff-Additionssalze von substituierten 1-Phenyl-2-triazolyl-1-penten-3-olen der Formel (I) mit Streckmitteln und/oder oberflächenaktiven Stoffen vermischt.

7) 1-Phenyl-2-triazolyl-1-penten-3-one der Formel

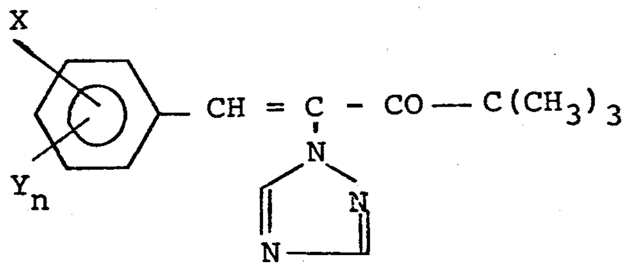

(II)

in welcher

X für 2-Trifluormethyl, 4-Trifluormethyl oder 3-Nitro steht,

Y für 2-Chlor, 3-Chlor oder 4-Chlor steht und

n für 0 oder 1 steht.

8) Verfahren zur Herstellung von 1-Phenyl-2-triazolyl-1-penten-3-onen der Formel

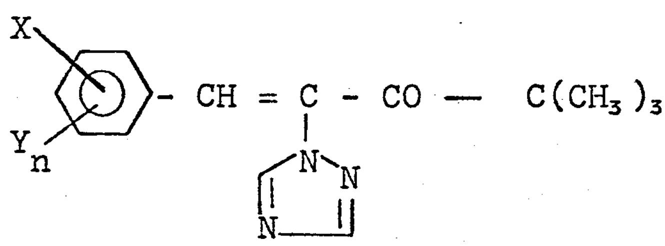

(II)

in welcher

X für 2-Trifluormethyl, 4-Trifluormethyl oder 3-Nitro steht,

Y für 2-Chlor, 3-Chlor oder 4-Chlor steht und

n für 0 oder 1 steht,

dadurch gekennzeichnet, daß man Triazolylpinakolin der Formel

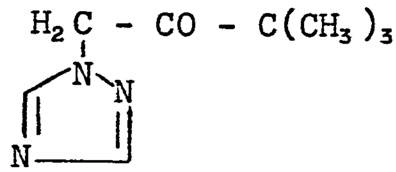

(III)

mit Aldehyden der Formel

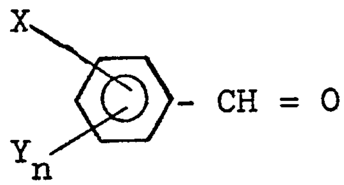

(IV)

in welcher

X, Y und n    die oben angegebene Bedeutung haben,

in Gegenwart eines Lösungsmittels und in Gegenwart eines Katalysators umsetzt.